# EUROPEAN PATENT APPLICATION

(11) **EP 1 175 915 A1**
(43) Date of publication of application: **30.01.2002**
(21) Application number: 01306421.7
(22) Date of filing: 26.07.2001
(51) Int. Cl.: A61K 47/48, A61K 31/405

(54) **A method for preparing resinates**

(30) Priority: 27.07.2000 US 221022 P
(71) Applicant: ROHM AND HAAS COMPANY, Philadelphia, Pennsylvania 19106-2399 (US)
(72) Inventor: Hughes, Lyn, Harleysville, Pennsylvania 19438 (US); Bellamy, Simon Andrew, Redhill, Surrey RH1 6DD (GB)
(74) Representative: Kent, Venetia Katherine

(57) **Abstract**

A highly productive and environmentally friendly method of loading pharmaceutically active substances onto ion exchange resins using water and, if desired, a water miscible or water-immiscible solvent.

## Description

### BACKGROUND OF THE INVENTION

The present invention relates to a method for the aqueous loading of poorly water soluble and soluble pharmaceutically active substances onto ion exchange resins.

It is well know in the art that using a complex formed between a polymeric material and an active substance can be beneficial. Such benefits can include changes in the release rate of drugs, taste masking of bitter drugs, control of the site of administration of drugs, control of the release of flavor substances, and stabilization of unstable substances.

The preparation of an active substance/ion exchange resin complex is called loading. The ion exchange resins complexed with the active substance are called resinates. The methods for loading have been varied, but in many cases are either problematic or limited in their application.

The typical method for loading active substances onto an ion exchange resin is to dissolve an acidic or basic, ionizable active substance in water, and then mix it with a suitable ion exchange resin. See, US2,990,332. The active substance is absorbed into the resin by the mechanism of ion exchange. The extent of loading will depend on several factors, including the rate of diffusion, the equilibrium constant, temperature, and the presence of other ions. The water is then removed by filtration, and the ion exchange resin dried by heating. As a general rule, anion exchange resins are useful for the loading of acidic substances, and cation exchange resins are useful for loading basic substances.

The need to dissolve the active substance to be loaded can lead to very large volumes of solution if the active substance has poor solubility in the loading medium. This leads to very low productivity in a commercial scale process. To overcome this problem, water miscible organic co-solvents such as ethanol are frequently used to increase the solubility and to reduce the total volume of solution. Introduction of these co-solvents into the process can add significant cost, because they are typically not recovered. They can increase the amount of hazardous waste generated, and introduce processing problems related to flammability and toxicity.

In the currently used commercial processes for making the resinates of active substances, said active substance is loaded onto a powdered, anion or cation ion exchange resin. The loading is performed in a predominantly aqueous system, whereby the active substance becomes immobilized on the resin by reaction with the functional groups of the resin. Use of an aqueous system for the loading has the disadvantage that the resulting slurry has to be dewatered and dried. This is currently achieved in a number of different ways, e.g., dewater in a decanter, and then dry in a vacuum dryer; or evaporate the water directly from the slurry in a vacuum distillation apparatus; and evaporate the water directly from the slurry using a spray dryer. There are problems associated with each of these methods. The decanter operation is made difficult because the ion exchange resin contains a significant fraction of very fine particles (<40 micron), and wet-cakes from such decanters can still contain >60% water by weight. The spray dryer and vacuum distillation operations are energy wasteful because all the water is removed by conversion to water vapor. Also, these methods can lead to particle agglomeration. Avoidance of these problems by using typical organic solvents leads to problems of toxicity from the residual solvent, safety problems from flammability, and environmental problems from vapor emissions and waste disposal.

The use of non-aqueous solvents as media for ion exchange reactions has been reported. See *"Ion Exchange Resins"* by Robert Kunin, p. 310, published by Robert E. Krieger Publishing Co, 1990. However, reaction times are reported to be very long for non-swelling solvents. Further, the solvents typically used are not optimum for industrial scale because they are flammable, or toxic, or difficult to remove efficiently, or difficult to re-use, or environmentally unacceptable, or high cost.

Many drug substances are hydrophobic and are poorly soluble in water. While this can be somewhat advantageous for absorption from solution into the gastrointestinal system, the actual dissolution of such drugs into physiological fluids can be very inefficient. This can result not only from a low solubility, but also a low rate of dissolution. This low rate of dissolution is itself the result of poor wettability of the hydrophobic solid, and the thermodynamic barrier caused by high crystal lattice energy which is difficult to overcome with water. This poor dissolution into physiological fluids can result in very poor and/or variable bioavailability of the drugs. Methods to improve the dissolution can thereby improve bioavailability.

A number of solutions have been explored, including grinding the drug to very small particle size (WO99/30687) and supplying it as a solution in oils (EP0306236B1). Each of these techniques has disadvantages. For example, not all drugs can be ground to very fine particle size due to low melting point or heat sensitivity. Dissolution in oils or dispersion in other matrices severely restricts the formulation options. There is a need for a method to improve dissolution that does not suffer these disadvantages.

The use of ion exchange resins to improve the rate of dissolution of weakly ionic compounds was reported by Irwin. *See,* Irwin, et al, Drug Deliv. and Ind. Pharm, 16(6), 883 (1990). Irwin observed faster dissolution of mefenamic acid from a powdered, strong base anion exchange resin when compared to a solid suspension. The loading method used by Irwin employed an aqueous medium as known to those skilled in the art.

Thus, there is a need in the art for an active ingredient loading method that is environmentally friendly, safe, low cost, and capable of high productivity. There is also need for a method that improves the dissolution of poorly soluble drugs that is not limited by melting point or temperature sensitivity, and is compatible with most existing formulation methods. Applicants have surprisingly discovered how to load poorly soluble or soluble active substances onto ion exchange resins using water, water miscible, and water immiscible solvents or mixtures thereof. Further, when said miscible and immiscible solvents are omitted, and only water is used, the amount of water needed is surprisingly very much less than that required to completely dissolve the active substance. Finally, Applicants have also unexpectedly discovered that the resinates of poorly soluble drugs made by the process of the present invention have a faster drug dissolution rate under physiological conditions.

The following terms have the following meanings herein:

The term "solubility," as used herein, means solubility as defined in the US Pharmacopoeia, 24, pg. 10. For the purposes of this invention the descriptor 'poorly soluble' will be used to describe substances that are very slightly soluble or practically insoluble in water by the USP definition. This solubility is <1 part of solute per 1000 parts of solvent. The descriptor 'soluble' will be used to describe substances with a solubility >1 part solute per 1000 parts solvent.

The term "water retention capacity" as used herein is used to describe the maximum amount of water that an ion exchange resin can retain within the polymer phase and in any pores. (ASTM D2187: Standard Test Methods for Physical and Chemical Properties of Particulate Ion Exchange Resin. Test Method B: Water Retention Capacity)

The term "resinate," as used herein, means an active substance/ion exchange resin complex.

The terms "loaded" and "loading" as used here-in mean the preparation of a resinate. The amount of loading means the amount of active substance incorporated into the resin to form a resinate.

Further, ion exchange resins are characterized by their capacity to exchange ions. This is expressed as the "Ion Exchange Capacity." For cation exchange resins the term used is "Cation Exchange Capacity," and for anion exchange resins the term used is "Anion Exchange Capacity." The ion exchange capacity is measured as the number equivalents of an ion that can be exchanged and can be expressed with reference to the mass of the polymer ( herein abbreviated to "Weight Capacity") or its volume (often abbreviated to "Volume Capacity"). A frequently used unit for weight capacity is "milliequivalents of exchange capacity per gram of dry polymer." This is commonly abbreviated to "meq/g."

Ion exchange resins are manufactured in different forms. These forms can include spherical and non-spherical particles with size in the range of 0.001mm to 2mm. The non-spherical particles are frequently manufactured by grinding of the spherical particles. Products made in this way typically have particle size in the range 0.001mm to 0.2mm. The spherical particles are frequently known in the art as 'Whole Bead.' The non-spherical particles are frequently known in the art as 'Powders.'

### STATEMENT OF THE INVENTION

The present invention relates to a method for preparing a resinate comprising the steps of:
a. blending a poorly water soluble or soluble active substance with an ion exchange resin and a solvent selected from the group consisting of water, a water miscible solvent, a water-immiscible solvent or mixtures thereof to form an active substance/resin/solvent mixture;
b. maintaining said mixture, at a pressure and temperature that maintains said mixture in the liquid state, for 1 second to 48 hours.

### DETAILED DESCRIPTION OF THE INVENTION

The present invention relates to a method for preparing a resinate comprising the steps of:
a. blending a poorly water soluble or soluble active substance with an ion exchange resin and a solvent selected from the group consisting of water, a water miscible solvent, a water-immiscible solvent or mixtures thereof to form an active substance/resin/solvent mixture;
b. maintaining said mixture, at a pressure and temperature that maintains said mixture in the liquid state, for 1 second to 48 hours.

Ion exchange resins useful in the practice of the present invention include, but are not limited to, anionic exchange resins and cationic exchange resins. Preferably, said resins are suitable for human and animal ingestion.

Preferred anionic exchange resins include, but are not limited to, styrenic strongly basic anion exchange resins with a quaternary amine functionality having a weight capacity of 0.1 to 15 meq/g, and styrenic weakly basic anion exchange resins with a primary, secondary, or tertiary amine functionality having a weight capacity of 0.1 to 8.5 meq/g, and acrylic or methacrylic strongly basic anion exchange resins with a quaternary amine functionality having a weight capacity of 0.1 to 12 meq/g, and acrylic or methacrylic weakly basic anion exchange resins with a primary, secondary, or tertiary amine functionality having a weight capacity of 0.1 to 12 meq/g, and allylic and vinylic weakly basic anion exchange resins with a primary, secondary, or tertiary amine functionality having a weight capacity of 0.1 to 24 meq/g, that are suitable for human and animal ingestion.

Most preferred anionic exchange resins include, but are not limited to, styrenic anion exchange resins with quaternary amine functionality with weight capacity of 0.1 to 6 meq/g and acrylic anion exchange resins with tertiary amine functionality with weight capacity of 0.1 to 12 meq/g, that are suitable for human and animal ingestion.

Preferred cationic exchange resins include, but are not limited to, styrenic strongly acidic cation exchange resins with sulfonic or phosphonic acid functionalities having a weight capacity of 0.1 to 8 meq/g; and styrenic weakly acidic cation exchange resins with carboxylic or phenolic acid functionalities having a weight capacity of 0.1 to 8.5 meq/g; and acrylic or methacrylic weakly acidic cation exchange resins with a carboxylic or phenolic acid functionality with a weight capacity of 0.1 to 14 meq/g, that are suitable for human and animal ingestion.

Most preferred cationic exchange resins include, but are not limited to, styrenic weakly acidic cation exchange resin with a phenolic functionality with a weight capacity of 0.1 to 8.5 meq/g; and a styrenic strongly acidic cation exchange resin with a sulfonic acid functionality with weight capacity of 0.1 to 8 meq/g, or a methacrylic weakly acidic cation exchange resin with a carboxylic acid functionality with weight capacity of 0.1 to 12 meq/g.

Ion exchange resins useful in this invention have a moisture content between 0% and the water retention capacity of said resin.

Ion exchange resins useful in this invention are in powder or whole bead form.

Strongly acidic and weakly acidic cation exchange resins useful in the practice of the present invention are in the acid form or salt form or partial salt form.

Strongly basic anion exchange resins useful in this invention are in the salt form.

Weakly basic anion exchange resins useful in this invention are in the free-base form or salt form.

Water soluble or poorly soluble active substances useful in the practice of the present invention include, but are not limited to, pharmaceutically active substances, vitamins, flavors and fragrances, that have acidic or basic ionizable groups.

Pharmaceutically active substances include, but are not limited to, indomethacin, salicylic acid, ibuprofen, sulindac, piroxicam, naproxen, timolol, pilocarpine, acetylcholine, dibucaine, thorazine, promazine, chlorpromazine, acepromazine, aminopromazine, perazine, prochlorperazine, trifluoroperazine, thioproperazine, reserpine, deserpine, chlorprothixene, tiotixene, haloperidol, moperone, trifluorperidol, timiperone, droperidol, pimozide, sulpiride, tiapride, hydroxyzine, chlordiazepoxide, diazepam, propanolol, metoprolol, pindolol, imipramine, amitryptyline, mianserine, phenelzine, iproniazid, amphetamines, dexamphetamines, fenproporex, phentermine, amfepramone, pemoline, clofenciclan, cyprodenate, aminorex, mazindol, progabide, codergoctine, dihydroergocristine, vincamone, citicoline, physostigmine, pyritinol, meclofenoxate, lansoprazole, nifedipine, risperidone, clarithromycin, cisapride, nelfinavir, midazolam, lorazepam, nicotine, ciprofloxacin, quinapril, isotretinoin, valcyclovir, acyclovir, delavirdin, famciclovir, lamivudine, zalcitabine, osteltamivir, abacavir, prilosec, omeprazole, prozac, zantac, lisinopril.

The preferred water insoluble or poorly soluble pharmaceutically active substances include, but are not limited to indomethacin, lansoprazole, nifedipine, risperidone, clarithromycin, cisapride, nelfinavir, midazolam, lorazepam, ciprofloxacin, quinapril, and isotretinoin.

The most preferred water insoluble or poorly soluble pharmaceutically active substances are indomethacin, nelfinavir, and midazolam.

Vitamins useful in the practice of the present invention include, but are not limited to, A, C, E, and K.

Flavors and fragrances useful in the practice of the present invention include, but are not limited to, vanillin, methyl salicylate, thymol, ethyl vanillin.

The preferred solvents useful in the practice of the present invention are selected from the group consisting of water, water miscible solvents, water immiscible solvents and mixtures thereof.

Water miscible solvents useful in the practice of the present invention include, but are not limited to, methanol, ethanol, isopropanol, n-propanol, acetone, dimethylformamide, tetrahydrofuran, dimethyl sulfoxide, dimethyl ether, and acetic acid.

The preferred water miscible solvents are ethanol, isopropanol, n-propanol, and dimethyl ether.

The most preferred water miscible solvent is ethanol.

Water immiscible solvents useful in the practice of the present invention include, but are not limited to hydrocarbons, halogenated hydrocarbons, ethers, ketones, and esters having boiling points, at atmospheric pressure between 100°C and -100°C.

The preferred water immiscible solvents are fluorinated hydrocarbon solvents having boiling points, at atmospheric pressure between 30°C and -100°C

The more preferred water immiscible solvents are:
trifluoromethane (CF₃H);
fluoromethane (CH₃F);
difluoromethane (CF₂H₂);
1,1-difluoroethane (CF₂HCH₃);
1,1,1-trifluoroethane (CF₃CH₃);
1,1,1,2-tetrafluroethane (CF₃CFH₂)
pentafluoroethane (CF₃CF₂H);
1,1,1,2,2-pentafluorpropane (CF₃CF₂CH₃);
1,1,1,2,3-pentafluorpropane (CF₃CFHCFH₂);
1,1,1,2,2,3-hexafluoropropane (CF₃CF₂CFH₂);
1,1,1,2,3,3-hexafluoropropane (CF₃CFHCF₂H);
1,1,1,3,3,3-hexafluropropane (CF₃CH₂CF₃);
1,1,2,2,3,3-hexafluoropropane (CF₂HCF₂CF₂H);
1,1,1,2,2,3,3-heptafluoropropane (CF₃CF₂CF₂);
1,1,1,2,3,3,3-heptafluoropropane (CF₃CFHCF₃);

The most preferred water immiscible solvent is 1,1,1,2-tetrafluoroethane (CF₃CFH₂), also known as TFE. This solvent has a boiling point of -26.5°C at atmospheric pressure, is of low toxicity, is non-flammable, and is non ozone depleting.

The preferred range of ratios of ion exchange resin to solvent is 1:1 to 1:1000, the more preferred range is 1:1.5 to 1:100, and the most preferred range is 1:2 to 1:5.

Preferably, the loading of active substance in the resinate of the present invention is 5-100% of the ion exchange capacity of the resin, more preferably it is 10-90% of the ion exchange capacity of the resin, and most preferably it is 15-80% of the ion exchange capacity of the resin.

The preferred pressure range for the practice of the present invention is 5 to 35,000 kPascals, the more preferred range is 100 to 5000 kPascals, and the most preferred range is 350 to 700 kPascals.

The preferred temperature range for the practice of the present invention is 10°C to 100°C, the more preferred range is 0°C to 80°C, and the most preferred range is 5°C to 30°C.

Preferably, the time to prepare a resinate of the present invention is from 1 second to 48 hours, more preferably from 5 minutes to 4 hours, and most preferably from 5 minutes to 30 minutes.

While Example 1 surprisingly illustrates that a poorly soluble drug can be loaded onto an ion exchange resin with less water than is required to completely dissolve said drug, the loading process takes about 2 hours and the mixture must be dewatered.

However, the addition of a water-immiscible or water miscible solvent as described hereinabove reduces the loading time to between 1 minute and 20 minutes, and eliminates the need to dewater the mixture. For example, in a preferred embodiment of the invention, the amount of water required is such that it does not exceed the water retention capacity of the ion exchange resin. In this way there is no separate water phase in the mixture. Because of the property of ion exchange resins to absorb water up to the water retention capacity the water can either be present in the ion exchange resin at the start of the process, or added as a separate ingredient to the mixture. The water immiscible solvent can be removed from the final mixture either by filtration, or by vaporization. The vaporization can be achieved by using heat, or by reducing the pressure, and providing a heat source to maintain the temperature of the solution between room temperature and the atmospheric pressure boiling point of said solvent. Specifically, the active substance, a suitable hydrated anion or cation exchange resin, and TFE are mixed at a pressure of about 520 kPascals to maintain said TFE in the liquid state. The mixture is stirred at room temperature for between 5 and 20 minutes. During this period the active substance rapidly loads onto the ion exchange resin, such that there is no solid active substance left in the mixture, and the amount of active substance dissolved in the TFE is insignificantly small. The TFE is then removed by reducing the pressure such that the TFE boils. The TFE vapor can be recovered either by using a condenser at less than the boiling point of the TFE, or by using a compressor and condenser. Both recovery methods are well known in the art. The TFE can then be re-used.

The ion exchange resin loaded with poorly soluble active substance prepared using TFE has very unexpectedly been found to exhibit an improved dissolution rate of said active substance over those made using the prior art as described in Irwin *et al*, Drug Deliv and Ind Pharm, 16(6), 883 (1990). The rate of dissolution of the active substance, prepared according to the present invention, under physiological conditions is greatly increased when compared to similar compositions made using the prior art. This is illustrated by Examples 7-10. In these examples, the poorly soluble drug indomethacin is loaded on a weakly basic anion exchange resin either by the method of this invention using TFE, or by using an aqueous ethanol solution to represent the prior art. The samples were tested, both with and without drying, in a dissolution test apparatus using simulated intestinal fluid. The data demonstrated that the resinates made by the method of the present invention released the indomethacin at a rate approximately double that of the materials made using the prior art.

The following non limiting examples illustrate the practice of the present invention.

### EXAMPLE 1 - Water-only loading:

Add 0.5 g of indomethacin, a poorly soluble active substance, and 1.5g of an acrylic anion exchange resin with tertiary amine functionality and a weight capacity between 5.8 and 6.2 meq/g, such as Amberlite IRA67, available from the Rohm and Haas Company, in its fully hydrated state to a 25 ml vial. Add 6 g of water to the mixture, close the vial and shake the mixture. After 2 hours the indomethacin will have disappeared and the ion exchange resin will be yellow. Drain the water from the mixture, to yield the wet resinate.

This experiment illustrates the very large reduction in required reaction volume achieved by the invention over the prior art. The solubility of indomethacin in water is 14ppm so that approximately 37kg of water would be required to completely dissolve the amount of indomethacin used in this example. For a commercial scale operation this decrease in required volume would represent a 6000 fold increase in productivity over the prior art.

### EXAMPLE 2 - TFE with dried resin

Into a vessel that can be evacuated and can operate at least 750 kPascals and is equipped with a stirrer, charge 1.3g of a finely ground acrylic anion exchange resin with tertiary amine functionality and a weight capacity between 5.8 and 6.2 meq/g that has been dried to <5% moisture, such as derived from the resin Amberlite IRA67, available from the Rohm and Haas Company. To the same vessel charge 1g of indomethacin. Evacuate the air from the vessel, and then introduce 50g of 1,1,1,2-tetrafluoroethane (TFE) so that at the end of the addition the pressure is approximately 520 kPascals and the temperature is 20°C, such that the TFE is in the liquid state. Stir the mixture for 120 minutes maintaining the temperature and pressure. At the end of this period, stop the stirrer and allow the mixture to stand for a few minutes. It will be noted that the resin, which is still white, will float to the surface of the TFE, and the undissolved indomethacin solid will sink to the bottom. These observations indicated that no significant loading has taken place.

### EXAMPLE 3 - TFE wet loading

Proceed as in Example 2, except add 1.7g of water to the mixture. This is sufficient water to hydrate the ion exchange resin, but not sufficient to form a separate liquid water layer. After stirring for 10minutes stop the stirrer and allow the mixture to stand for a few minutes. It will be noted that the resin, now yellow in color, will float to the surface, and that there will be no indomethacin on the bottom of the vessel. Carefully remove approximately one half of the TFE as a liquid sample, without including any of the resinate. Remove the TFE from this sample by evaporation. It will be noted that there is no significant solid residue left after the TFE has been removed. These observations indicate that all the indomethacin loaded onto the resin.

### EXAMPLE 4 - Dichlorethane loading

Proceed as in Example 1, except use 7g of dichloroethane. After shaking for 10 minutes, it will be noted that the resin is now yellow, and that there is no solid indomethacin present. This observation indicates the indomethacin loaded onto the ion exchange resin.

### EXAMPLE 5 - Pentane loading

Proceed as in Example 1, except use 3.5g of pentane instead of dichloroethane. After shaking for 20 minutes, it will be noted that the resin is now yellow, and that there is no solid indomethacin present. This observation indicates the indomethacin loaded onto the ion exchange resin

### EXAMPLE 6 - Preparing a resinate of nelfinivir and Amberlite IRP64

The same as Example 3, except use 1g of Nelfinivir, 1.4g of water, and 1.6g of a dried, ground methacrylic weakly acidic cation exchange resin with carboxylic acid functionality with weight capacity between 10.1 and 11.1 meq/g (such as Amberlite IRP64, available from the Rohm and Haas Company).

Dissolution testing samples were prepared accordingly:

### EXAMPLE 7 - Preparation of the sample of the present invention for dissolution testing.

In the same equipment as used in Example 2, charge 3g of an acrylic anion exchange resin with tertiary amine functionality and a weight capacity between 5.8 and 6.2 meq/g, such as Amberlite IRA67, available from the Rohm and Haas Company, in its fully hydrated state, whole bead form. To the same vessel charge 1g of indomethacin. Evacuate the air from the vessel, and then introduce 50g of 1,1,1,2-tetrafluoroethane (TFE) so that at the end of the addition the pressure is approximately 520 kPascals and the temperature is 20°C, such that the TFE is in the liquid state. Stir the mixture at room temperature for 10 minutes. During this period the resin will change to a yellow color, indicating indomethacin loading. Reduce the pressure in the loading vessel by venting it to the atmosphere to remove the TFE. There remains a water-wet resinate, that is indomethacin loaded onto the anion exchange resin.

### EXAMPLE 8 - Preparation of the sample of the present invention for dissolution testing.

Proceed as in Example 7, except dry the resinate in a vacuum oven at 60°C for 4 hours.

### EXAMPLE 9 - Preparation of a sample of the prior art for dissolution testing.

Prepare a solution of 1g of indomethacin in 200ml of 50% aqueous ethanol. To this add 3g of an acrylic anion exchange resin with tertiary amine functionality and a weight capacity between 5.8 and 6.2 meq/g (such as Amberlite IRA67, available from Rohm and Haas Company, Philadelphia, Pennsylvania) in its fully hydrated state, whole bead form. Shake the mixture overnight at room temperature. During this period the yellow solution will lose most of its color, and the resin will become yellow. Drain the solution from the mixture, and analyze it for indomethacin using a uv/vis spectrometer at a wavelength of 318nm, such as described in US Pharmacopoeia, USP24 p. 874. The analysis will indicate approximately 0.1g of the indomethacin was left in solution, and did not load onto the resin.

### EXAMPLE 10 - Preparation of a sample of the prior art for dissolution testing.

Proceed as in Example 9, except dry the resinate in a vacuum oven at 60°C for 4 hours.

Dissolution testing performed on Examples 7-10.

For samples of each of the Examples 7-10, weigh out sufficient resinate to give 25mg of indomethacin. Add the resinate to 750ml of Simulated Intestinal Fluid TS, as defined by USP24, except that no purified pancreatin is included, at room temperature. Stir the mixture at 250rpm and take samples at 0, 10, 20, 45, and 120 minutes. Analyze the samples for indomethacin using uv/vis spectrometry. The data obtained is illustrated in TABLE 1 below:

**TABLE 1-**

| **% Release of Indomethacin** | | | | | |
|---|---|---|---|---|---|
| **Time, (mins)** | **0** | **10** | **20** | **45** | **120** |
| Example 7 | 0 | 12.1 | 14.7 | 23.2 | 35.0 |
| Example 8 | 0 | 14.6 | 18.3 | 28.5 | 41.6 |
| Example 9 | 0 | 8.8 | 8.8 | 14.3 | 25.5 |
| Example 10 | 0 | 7.3 | 7.3 | 12.1 | 22.0 |

While not intending to be bound by theory, microscopic examination of the resinate from Examples 7 and 8, as compared with Examples 9 and 10, reveals that the increase in the rate of dissolution of the active ingredient is caused by an anisotropic distribution of the active ingredient in the resinate particles. This distribution is such that there is a higher concentration of active ingredient on and near the surface of the particle than there is deeper within the particle. This reduces the average distance (diffusion path) that a molecule has to diffuse before it reaches the surface, at which point it dissolves into the bulk liquid phase. This reduction in the diffusion path results in faster overall release of the active ingredient. The anisotropic distribution is a direct result of the loading method, which produces a very high localized concentration of active substance at the particle surface, such that diffusion into the particle is not fast enough to give isotropic distribution.

### Example 11 - Use of a water miscible solvent

The same as Example 1 except that the instead of adding water, add 2.5g of water and 2.5g of ethanol. The indomethacin will load within 2 hours. The supernatant at the end of the experiment will contain approximately 0.003g of indomethacin that did not load.

## Claims

1. A method for preparing a resinate comprising the steps of:
a. blending a poorly water soluble or soluble active substance with an ion exchange resin and a solvent selected from the group consisting of water, a water miscible solvent, and a water-immiscible solvent or mixtures thereof to form an active substance/resin/solvent mixture;
b. maintaining said mixture, at a pressure and temperature that maintains said mixture in the liquid state, for 1 second to 48 hours.

2. A method according to Claim 1 wherein the ratio of resin to solvent is 1:1 to 1:1000.

3. A method according to Claim 2, wherein the ratio of resin to solvent is 1:1.5 to 1:100.

4. A method according to Claim 3, wherein the ratio of resin to solvent is 1:2 to 1:5.

5. A method according to Claim 3, wherein the active substance is loaded at 5-100% of the ion exchange capacity of the resin.

6. A method according to Claim 3, wherein the active substance is loaded at 10-90% of the ion exchange capacity of the resin.

7. A method according to Claim 3, wherein the active substance is loaded at 15-80% of the ion exchange capacity of the resin.

8. A method according to Claim 1 wherein the water immiscible solvent is a hydrocarbon, halogenated hydrocarbon, ether, ketone, or ester, mixture thereof, having a boiling point, at atmospheric pressure between 100°C and -100°C.

9. A method according to Claim 8 wherein the water immiscible solvent is a fluorinated hydrocarbon having a boiling point, at atmospheric pressure between 30°C and -100°C.

10. A method according to Claim 9 wherein the water immiscible solvent is 1,1,1,2-tetrafluoroethane.

11. A method according to Claim 1 wherein the ion exchange resin is an anionic exchange resin or cationic exchange resin.

12. A method according to Claim 11, wherein the ion exchange resin is a cationic exchange resin.

13. A method according to Claim 11, wherein the ion exchange resin is an anionic exchange resin.

14. A method according to Claim 1 wherein the water miscible solvent is a selected from the group consisting of ethanol, isopropanol, n-propanol and dimethyl ether.

15. A method for administering a poorly soluble medicament comprising administering an effective amount of a resinate prepared according to Claim 1.
